# EUROPEAN PATENT APPLICATION

(11) **EP 4 660 195 A1**
(43) Date of publication of application: **10.12.2025**
(21) Application number: 24382612.0
(22) Date of filing: 05.06.2024
(51) Int. Cl.: C07D 417/14, A61P 7/04, A61K 31/496

(54) **CRYSTALLINE FORMS OF AVATROMBOPAG MALEATE AND PREPARATION THEREOF**

(71) Applicant: NOUCOR HEALTH S.A., Palau-solità i Plegamans 08184 (Barcelona) (ES)
(72) Inventor: SANTOS RAMOS, Javier, 08202 Sabadell, Bacelona (ES); CARRASQUER LOZANO, Laura, 08480 L'Ametlla del Valles, Barcelona (ES); MARTÍNEZ PÉREZ, Oscar, 08224 Terrassa (ES); RAMÍREZ ARTERO, Jesús, 43130 Tarragona (ES); CERÓN BERTRAN, Jordi Carles, 43761 La Pobla de Montornès, Tarragona (ES)
(74) Representative: ABG Intellectual Property Law, S.L.

(57) **Abstract**

The present invention relates to crystalline forms of Avatrombopag maleate, to a preparation method thereof, to pharmaceutical compositions comprising said solid forms and to the use thereof in medicine, particularly for increasing the level of platelets in a patient. The invention also relates to the use of said solid form in the prevention or treatment of thrombocytopenia or chronic liver disease.

## Description

### FIELD OF THE INVENTION

The present invention relates to crystalline forms of Avatrombopag maleate, to a preparation method thereof, to pharmaceutical compositions comprising said solid forms and to the use thereof in medicine, particularly for increasing the level of platelets in a patient. The invention also relates to the use of said solid form in the prevention or treatment of thrombocytopenia or chronic liver disease.

### BACKGROUND

1 -[3-Chloro-5-[[[4-(4-chloro-2-thienyl)-5-(4-cyclohexyl-1 -piperazinyl)-2-thiazolyl]amino]carbonyl]-2-pyridinyl]-4-piperidinecarboxylic acid, also named Avatrombopag is an active ingredient having as molecular formula 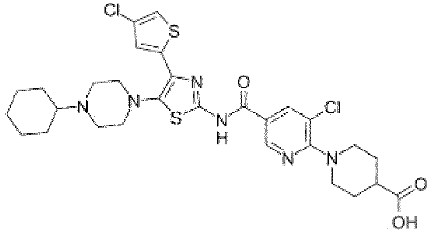 This compound acts as a thrombopoietin receptor (c-Mpl) agonist and is suitable for increasing the number of platelets in a patient without increasing platelet activation. This active ingredient is commercialized as a maleate salt under the commercial name Doptelet^{®}. The drug is typically formulated as tablets comprising an amount of a maleate salt of Avatrombopag equivalent to 20 mg of Avatrombopag and a pharmaceutically acceptable excipient such as lactose.

Avatrombopag maleate has been specifically disclosed in WO 2004/029049 A1. The disclosed process for the preparation of Avatrombopag maleate as a crystalline solid comprises re-crystallization in ethanol 80%. This document discloses that the resulting crystalline solid presents a Differential Scanning Calorimetry diagram showing endothermic peaks near 198 °C and 271 °C. In addition, according to the US Food and Drug Administration, the aqueous solubility of Avatrombopag maleate at various pH levels indicates that the drug substance is practically insoluble at pH 1 to 11. In addition, the major absorption site of Avatrombopag in the gastric system is duodenum (pH ca. 6) followed by jejunum (pH of about 7-8), while the absorption of the drug in the stomach (pH of 1.5 - 2) is minimal. To the extent the bioavailability of a drug is linked to its solubility, several ways of improving the solubility of Avatrombopag maleate have been explored. In this regard, several solid forms of Avatrombopag maleate have been disclosed in the art.

In patent application WO 2013/018362 A1, polymorphism of Avatrombopag maleate is disclosed. According to the authors, form A, which is the form disclosed in WO 2004/029049 A1 is susceptible of morphing to a further crystalline form upon production at the large scale following the production method disclosed in WO 2004/029049 A1. To the extent a drug must meet requirements of constant quality and effects, the authors conclude that the crystalline form A is inappropriate and further provide two crystalline forms of Avatrombopag maleate, forms B and C, from B being obtained as a derivative of form A obtained during synthesis and form C resulting from the formation of the Avatrombopag maleate salt in a heated mixture of DMSO, acetone and water. According to this document, crystal forms A and C type crystal possess excellent oral absorption, but crystal form B exhibits inferior oral absorption. This document thus clearly designates the crystal form C as the only crystal form of Avatrombopag maleate having both large-scale manufacturability and oral availability.

Patent specification IN 429420 B discloses a process for the manufacture of the crystal forms B and C of Avatrombopag maleate disclosed in WO 2013/018362 A1 from an amorphous solid form of Avatrombopag maleate. Said amorphous solid form is obtainable by a process comprising the alkaline hydrolysis of an ethyl ester of Avatrombopag to form the free base form of Avatrombopag, followed by the addition of maleic acid, thus producing an amorphous form of the maleate salt of Avatrombopag. Said amorphous form may further be used for the preparation of crystalline from B, by heating in water or ethanol, or of crystalline form C, by suspension in a ketone solvent. Further procedures for the preparation of solid form C of Avatrombopag maleate have been reported in the art, as disclosed for instance in CN 112409350 A and CN 106749226 A. CN 112409350 A discloses in particular the preparation of solid form C by drying of a new solid form D, which is a solvate of form C.

In patent application WO 2020/044364 A1, the authors disclose further solid forms of Avatrombopag maleate, among which an amorphous form of Avatrombopag maleate, crystalline Form M2 of Avatrombopag, crystalline Form M3 of Avatrombopag, crystalline Form M4 of Avatrombopag, crystalline Form M5 of Avatrombopag, crystalline Form M1 of Avatrombopag maleate, crystalline Form M2 of Avatrombopag maleate, crystalline Form M3 of Avatrombopag maleate, amorphous Avatrombopag, amorphous Avatrombopag maleate, amorphous solid dispersions of Avatrombopag maleate and processes for the preparation thereof. However, this patent is silent about the oral absorption of the proposed solid-state forms as well as if they are commercially suitable for industrial scale up.

CN 109111440 A discloses a further solid crystalline form of Avatrombopag in its free acid form, which has the characteristics of good stability, low hygroscopicity and convenient long-term storage of drugs. The authors are however silent about the solubility of said solid form and about the oral bioavailability of the proposed crystalline form as well as if it is commercially suitable for industrial scale up.

In a different approach, CN 114377147 A discloses inclusion complexes of Avatrombopag maleate with sulfonylated or hydroxylated cyclodextrin derivatives exhibiting improved solubility at pH 6.8 than solid dispersions of Avatrombopag maleate.

From what is disclosed in the art, it derives that there is still a need for solid forms of Avatrombopag maleate with improved oral bioavailability and industrial scalability.

### SUMMARY OF THE INVENTION

After exhaustive research, the inventors have developed a solid form of Avatrombopag maleate with improved oral bioavailability. The solid form of Avatrombopag maleate, also referred to herein as "crystalline form alpha", advantageously exhibits an improved solubility in water in acidic pH, such as gastric pH (pH of 1-2). This results in a larger portion of Avatrombopag maleate being dissolved in the stomach when administered orally to a patient. Thus, advantageously, the solid form of Avatrombopag maleate of the invention has an improved oral bioavailability over the solid forms of Avatrombopag maleate disclosed in the art as well as the potential to provide a therapeutic effect earlier than the solid forms of Avatrombopag maleate disclosed in the art, as it is believed that the plasma concentration of avatrombopag increases earlier after oral administration of avatrombopag as a result of the higher solubility of avatrombopag in gastric pH. in addition to the above, said crystalline form alpha is thermally stable and can be produced at large scale. As a further advantage, the observed improved solubility also broadens the scope of manufacturing operations which can be performed with the solid form alpha of Avatrombopag maleate. In this regard, the solid form alpha of Avatrombopag maleate is particularly suitable for industrial manufacturing.

The crystalline form Avatrombopagalpha of Avatrombopag maleate may be obtained by desolvation of a second crystalline solid form of Avatrombopag maleate, also referred to herein as "crystalline form beta", which also forms part of the invention. Said crystalline form of Avatrombopag maleate beta may further derive from of a third crystalline solid form of Avatrombopag maleate, also referred to herein as "crystalline form gamma", which also forms part of the invention.

Thus, a first aspect of the invention relates to a crystalline form of Avatrombopag maleate that is selected from the group consisting of:
(i) a crystalline form alpha characterized in that its powder X-Ray diffraction spectrum measured using a CuKα radiation comprises diffraction peaks at least at the following 2 θ values of 9.1 ± 0.2 degrees; 10.3 ± 0.2 degrees; 16.0 ± 0.2 degrees; 19.0 ± 0.2 degrees; 22.8 ± 0.2 degrees and 28.3± 0.2 degrees;
(ii) a crystalline form beta characterized in that its powder X-Ray diffraction spectrum measured using a CuKα radiation comprises diffraction peaks at least at the following 2 θ values of 5.6 ± 0.2 degrees; 14.8 ± 0.2 degrees; 18.7 ± 0.2 degrees; 20.5 ± 0.2 degrees; 22.3 ± 0.2 degrees and 26.8 ± 0.2 degrees;
(iii) a crystalline form gamma characterized in that its powder X-Ray diffraction spectrum measured using a CuKα radiation comprises diffraction peaks at least at the following 2 θ values of 5.7 ± 0.2 degrees; 14.1 ± 0.2 degrees; 17.7 ± 0.2 degrees; 18.7 ± 0.2 degrees; 20.7 ± 0.2 degrees; and 22.3 ± 0.2 degrees; and
(iv) a mixture thereof.

The second aspect of the invention relates to a pharmaceutical composition comprising a therapeutically effective amount of the crystalline form of Avatrombopag maleate alpha as defined in the first aspect of the invention and a pharmaceutically acceptable excipient.

The crystalline form of Avatrombopag maleate may be used in medicine, in particular for the treatment or prevention of thrombocytopenia or chronic liver disease.

The third aspect of the invention relates to the crystalline form of Avatrombopag maleate alpha as defined in the first aspect of the invention or to a pharmaceutical composition as defined in the second aspect of the invention for use in medicine.

The fourth aspect of the invention relates to the crystalline form of Avatrombopag maleate alpha as defined in the first aspect of the invention or to a pharmaceutical composition as defined in the second aspect of the invention for use in the treatment or prevention of thrombocytopenia or chronic liver disease.

The fifth aspect of the invention relates to a process for the preparation of a composition comprising a crystalline form alpha as defined in the first aspect of the invention comprising the steps of:
(i) providing a crystalline form of Avatrombopag maleate that is selected from the group consisting of a crystalline form gamma as defined in the first aspect of the invention, a crystalline form beta as defined in the first aspect of the invention and a mixture thereof;
(ii) desolvating the crystalline form of Avatrombopag maleate provided in step (i); or; alternatively,
(iii) contacting an amount of Avatrombopag with a substantially equimolar amount of maleic acid in methanol to provide a suspension;
(iv) isolating the solid resulting from step (iii); and
(v) drying the solid resulting from step (iv) under vacuum and at a temperature of between 60 °C and 160 °C.

### BRIEF DESCRIPTION OF THE DRAWINGS

Fig. 1 shows the Powder X-Ray Diffraction (PXRD) pattern as measured using a CuKα radiation of crystalline form alpha of the first aspect of the invention.
Fig. 2 shows the Powder X-Ray Diffraction (PXRD) pattern as measured using a CuKα radiation of crystalline form beta of the first aspect of the invention.
Fig. 3 shows the Powder X-Ray Diffraction (PXRD) pattern as measured using a CuKα radiation of crystalline form gamma of the first aspect of the invention.
Fig. 4 shows (top) the thermal gravimetric analysis diagram of crystal form alpha and (bottom) the Differential Scanning Calorimetry profile of crystal form alpha performed as described herein.
Fig. 5 shows (top) the thermal gravimetric analysis diagram of crystal form beta and (bottom) the Differential Scanning Calorimetry profile of crystal form beta performed as described herein.
Fig. 6 shows the isotherm of water sorption (plain line) and desorption (dashed line) of crystalline form alpha as measured by Dynamic Vapor Sorption Analysis as disclosed in the Examples; y axis shows the weight variation of the solid form, expressed as a percentage and x axis shows the relative humidity.
Fig. 7 shows the PXRD patterns of solid form alpha before (bottom) and after (top) of the Dynamic Vapor Sorption Analysis.
Fig. 8 shows the PXRD patterns of (a) crystal solid form alpha and (b) crystal solid form alpha placed at 40 °C and 75% relative humidity after a period of (b) 1 day, (c) 5 days, (d) 13 days.
Fig. 9 shows the PXRD patterns of (a) crystal solid form alpha and crystal solid form alpha placed at 90 °C for 24 hours in (b) an open vial or (c) a closed vial.
Fig. 10 shows the ¹H NMR spectra of (a) crystal solid form alpha and crystal solid form alpha placed at 90 °C for 24 hours in (b) an open vial or (c) a closed vial.
Fig. 11 shows the calibration curve established for determining the solubility of solid forms of Avatrombopag maleate, y axis shows the area under the curve of the UV-Vis spectrum of the absorbance peak at 210 nm and the x axis shows the concentration of Avatrombopag maleate in a stock solution.

### DETAILED DESCRIPTION

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

For the purposes of the invention, any ranges given include both the lower and the upper end-points of the range. Ranges given, such as temperatures, times, molar ratio, volume ratio and the like, should be considered approximate (i.e. with a 5% margin of variation around indicated point), unless specifically stated.

An "effective amount" or "therapeutically effective amount" of a drug or pharmacologically active agent means a non-toxic but sufficient amount of the drug or agent to provide the desired effect. The amount that is "effective" will vary from subject to subject, depending on the age and general condition of the individual, the particular active agent or agents, and the like. Therefore, it is not always possible to specify an exact "effective quantity". However, an appropriate "effective" amount in any individual case can be determined by the skilled person using routine experimentation.

As used herein, the term "treatment" or derivations thereof include the eradication, removal, reversion, alleviation, modification, or control of a disease or disorder.

As used herein, the term "prevention" or derivations thereof refer to the avoiding or minimizing of the onset or development of a disease or disorder.

The term "excipient" refers to components of a drug compound other than the active ingredient. They preferably include a "carrier, adjuvant and/or vehicle". Carriers are forms to which substances are incorporated to improve the delivery and the effectiveness of drugs. Drug carriers are used in drug-delivery systems such as the controlled-release technology to prolong *in vivo* drug actions, decrease drug metabolism, and reduce drug toxicity. Carriers are also used in designs to increase the effectiveness of drug delivery to the target sites of pharmacological actions. Adjuvant is a substance added to a drug product formulation that affects the action of the active ingredient in a predictable way. Vehicle is an excipient or a substance, preferably without therapeutic action, used as a medium to give bulk for the administration of medicines. Such pharmaceutical carriers, adjuvants or vehicles can be sterile liquids, such as water and oils, including those of petroleum, animal, vegetable or synthetic origin, such as peanut oil, soybean oil, mineral oil, sesame oil and the like, excipients, disgregants, wetting agents or diluents.

As defined above, a first aspect of the invention relates to a crystalline form of Avatrombopag maleate that is selected from the group consisting of:
(i) a crystalline form alpha characterized in that its powder X-Ray diffraction spectrum measured using a CuKα radiation comprises diffraction peaks at least at the following 2 θ values of 9.1 ± 0.2 degrees; 10.3 ± 0.2 degrees; 16.0 ± 0.2 degrees; 19.0 ± 0.2 degrees; 22.8 ± 0.2 degrees and 28.3± 0.2 degrees;
(ii) a crystalline form beta characterized in that its powder X-Ray diffraction spectrum measured using a CuKα radiation comprises diffraction peaks at least at the following 2 θ values of 5.6 ± 0.2 degrees; 14.8 ± 0.2 degrees; 18.7 ± 0.2 degrees; 20.5 ± 0.2 degrees; 22.3 ± 0.2 degrees and 26.8 ± 0.2 degrees;
(iii) a crystalline form gamma characterized in that its powder X-Ray diffraction spectrum measured using a CuKα radiation comprises diffraction peaks at least at the following 2 θ values of 5.7 ± 0.2 degrees; 14.1 ± 0.2 degrees; 17.7 ± 0.2 degrees; 18.7 ± 0.2 degrees; 20.7 ± 0.2 degrees; and 22.3 ± 0.2 degrees; and
(iv) a mixture thereof.

As mentioned above, crystal forms beta and gamma are precursor forms of crystal form alpha; crystal form gamma being itself a precursor of crystal form beta. In a preferred embodiment, the first aspect of the invention thus relates to a solid form of Avatrombopag maleate having the crystalline form alpha, optionally mixed with the crystalline form beta. In another preferred embodiment, the first aspect of the invention thus relates to a solid form of Avatrombopag maleate having the crystalline form alpha, optionally mixed with the crystalline form gamma. It is however preferred that the solid form of Avatrombopag maleate has the crystalline form alpha.

In another preferred embodiment, the first aspect of the invention relates to a solid form of Avatrombopag maleate having the crystalline form beta, optionally mixed with the crystalline form gamma. In said embodiment, it is however preferred that the solid form of Avatrombopag maleate has the crystalline form beta.

In another preferred embodiment, the first aspect of the invention relates to a solid form of Avatrombopag maleate having the crystalline form gamma.

It is also contemplated that the first aspect of the invention relates to a crystalline form of Avatrombopag maleate that is a mixture of crystal forms alpha, beta and gamma.

In preferred embodiments of the first aspect of the invention, the powder X-ray diffraction spectrum of the crystalline form alpha measured using a CuKα radiation further comprises diffraction peaks at least at the following 2 θ values of 6.6 ± 0.2 degrees, 13.1 ± 0.2 degrees; 17.0 ± 0.2 degrees; 18.4 ± 0.2 degrees; 20.5 ± 0.2 degrees and 23.2 ± 0.2 degrees.

In even more preferred embodiments of the first aspect of the invention, the powder X-ray diffraction spectrum of the crystalline form alpha measured using a CuKα radiation further comprises diffraction peaks at one or more, preferably all, of the following 2 θ values of 7.6 ± 0.2 degrees, 10.4 ± 0.2 degrees, 10.6 ± 0.2 degrees, 11.0 ± 0.2 degrees, 12.5 ± 0.2 degrees, 14.4 ± 0.2 degrees, 15.9 ± 0.2 degrees, 16.4 ± 0.2 degrees, 16.7 ± 0.2 degrees, 17.6 ± 0.2 degrees, 18.0 ± 0.2 degrees, 18.1 ± 0.2 degrees, 19.4 ± 0.2 degrees, 19.8 ± 0.2 degrees, 21.3 ± 0.2 degrees, 22.1 ± 0.2 degrees, 23.5 ± 0.2 degrees, 23.9 ± 0.2 degrees, 24.6 ± 0.2 degrees, 25.2 ± 0.2 degrees, 26.4 ± 0.2 degrees, 26.8 ± 0.2 degrees, 27.1 ± 0.2 degrees, 27.8 ± 0.2 degrees, 29.2 ± 0.2 degrees, 30.7 ± 0.2 degrees, 31.3 ± 0.2 degrees, 31.7 ± 0.2 degrees, 32.4 ± 0.2 degrees, 34.2 ± 0.2 degrees, 34.8 ± 0.2 degrees, 35.7 ± 0.2 degrees, 36.2 ± 0.2 degrees, 36.9 ± 0.2 degrees, 37.5 ± 0.2 degrees, 38.6 ± 0.2 degrees and 39.0 ± 0.2 degrees.

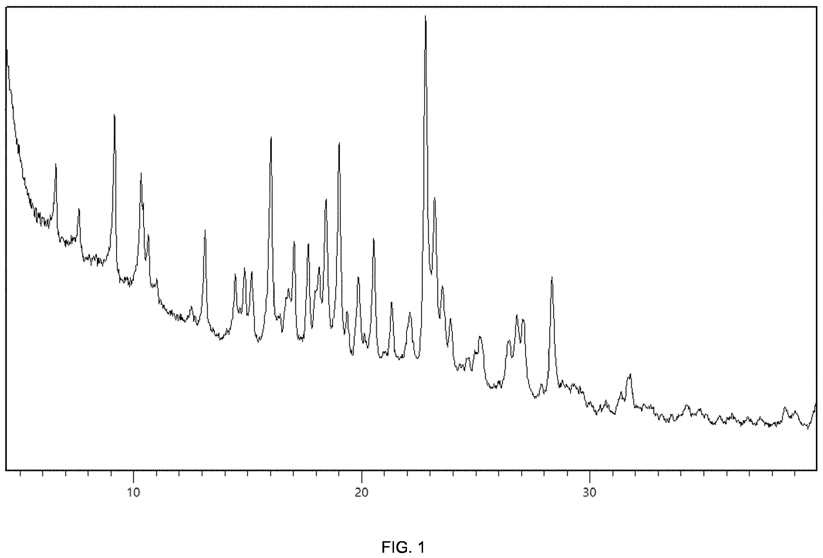

In even more preferred embodiments of the first aspect of the invention, the powder X-ray diffraction spectrum of the crystalline form alpha is substantially as shown in Figure 1.

The inventors have found that crystalline form alpha exhibits high thermal stability. In particular, neither the PXRD pattern of crystalline form alpha or the ¹H NMR spectrum is not substantially modified when the solid form is heated at 90 °C for 24 hours upon exposure to air. The inventors have also found that the PXRD pattern of the crystalline form alpha is not substantially modified when a sample of said solid form is exposed to an atmosphere of 75% relative humidity and a temperature of 40 °C for periods of 13 days. Additionally, the crystalline form alpha of Avatrombopag maleate exhibits a very low hygroscopicity, which makes it easy to handle and facilitates the preparation of final dosage forms.

In further preferred embodiments of the first aspect of the invention, the crystalline form alpha is further characterized in that it presents a diagram of Differential Scanning Calorimetry which comprises a first endothermic peak with an onset temperature of about 184 °C ± 2 °C, a second endothermic peak with an onset temperature of about 244 °C ± 2 °C and a third endothermic peak with an onset temperature of about 293 °C ± 2 °C; preferably, said diagram of Differential Scanning Calorimetry further comprises a first exothermic peak with an onset temperature of about 196 °C ± 2 °C and a second exothermic peak with an onset temperature of about 210 °C ± 2 °C.

In further preferred embodiments of the first aspect of the invention, in the diagram of Differential Scanning Calorimetry of the crystalline form alpha, the first endothermic peak presents an associated heat of about - 62 J/g ± 2 J/g, the second endothermic peak presents an associated heat of about - 56 J/g ± 2 J/g and/or the third endothermic peak presents an associated heat of about - 63 J/g ± 2 J/g; preferably, when said diagram comprises a first and a second exothermic peak, the first exothermic peak presents an associated heat of about 9 J/g ± 2 J/g and the second exothermic peak presents an associated heat of about 12 J/g ± 2 J/g.

In further preferred embodiments of the first aspect of the invention, the crystalline form alpha is further characterized in that the thermogravimetric analysis measured between 30 °C and 300 °C with a heating ramp of 10 °C per minute presents a weight loss of about 4% ± 0.2% between 160 °C and 208 °C and, optionally, a further weight loss of about 9% ± 0.2% between 208 °C and 300 °C.

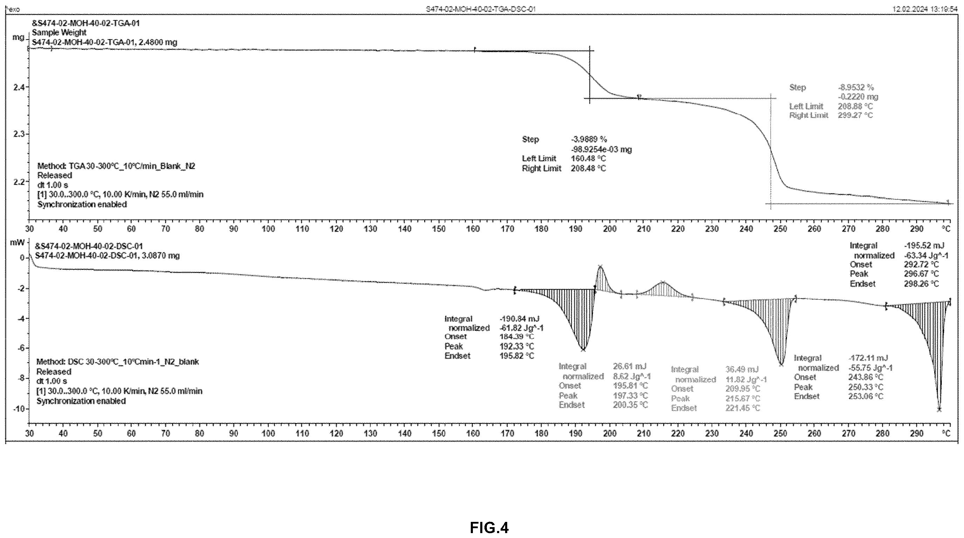

In further preferred embodiments of the first aspect of the invention, the crystalline form alpha is one having substantially the thermal gravimetric analysis and Differential Scanning Calorimetry analysis of Figure 4.

In further preferred embodiments of the first aspect of the invention, the crystalline form alpha is one which absorbs less than 0.7% of its weight of water when exposed to a relative humidity of 90%. This is advantageous as the crystalline form alpha is not substantially hygroscopic.

In further preferred embodiments of the first aspect of the invention, the crystalline form alpha is one having a thermodynamic solubility of at least 0.4 mg/mL when a sample of 100 mg of said crystalline form is placed in a solution having a pH of 1.2 for a period of 24 hours. Such solubility is advantageously higher than other solid forms known in the art, such as solid form B or solid form C.

In further preferred embodiments of the first aspect of the invention, the crystalline form alpha is one having a thermodynamic solubility of at least 0.06 mg/mL when a sample of 100 mg of said crystalline form is placed in a solution having a pH of 1.2 for a period of 1 hour.

In further preferred embodiments of the first aspect of the invention, the crystalline form alpha is one having a thermodynamic solubility of at least 0.4 mg/mL when a sample of 100 mg of said crystalline form is placed in a solution having a pH of 4.5 for a period of 24 hours.

In further preferred embodiments of the first aspect of the invention, the crystalline form alpha is substantially anhydrous.

in further preferred embodiments of the first aspect of the invention, the crystalline form alpha is one having a thermodynamic solubility of at least 0.03 mg/mL when a sample of 100 mg of said crystalline form is placed in a solution having a pH of 4.5 for a period of 1 hour.

In further preferred embodiments of the first aspect of the invention, the crystalline form alpha is one having a thermodynamic solubility of at least 0.25 mg/mL when a sample of 100 mg of said crystalline form is placed in a solution having a pH of 6.8 for a period of 24 hours.

In further preferred embodiments of the first aspect of the invention, the crystalline form alpha is one having a thermodynamic solubility of at least 0.025 mg/mL when a sample of 100 mg of said crystalline form is placed in a solution having a pH of 6.8 for a period of 1 hour.

In other preferred embodiments of the first aspect of the invention, the powder X-ray diffraction spectrum of the crystalline form beta measured using a CuKα radiation further comprises diffraction peaks at least at the following 2 θ values of 9.0 ± 0.2 degrees, 11.0 ± 0.2 degrees; 13.8 ± 0.2 degrees; 17.7 ± 0.2 degrees; 22.6 ± 0.2 degrees and 23.7 ± 0.2 degrees.

In even more preferred embodiments of the first aspect of the invention, the powder X-ray diffraction spectrum of the crystalline form beta measured using a CuKα radiation further comprises diffraction peaks at one or more, preferably all, of the following 2 θ values of 7.7 ± 0.2 degrees, 9.8 ± 0.2 degrees, 10.2 ± 0.2 degrees, 10.4 ± 0.2 degrees, 11.7 ± 0.2 degrees, 14.1 ± 0.2 degrees, 16.4 ± 0.2 degrees, 17.2 ± 0.2 degrees, 19.2 ± 0.2 degrees, 19.7 ± 0.2 degrees, 21.0 ± 0.2 degrees, 21.3 ± 0.2 degrees, 21.4 ± 0.2 degrees, 21.8 ± 0.2 degrees, 24.3 ± 0.2 degrees, 25.4 ± 0.2 degrees, 26.0 ± 0.2 degrees, 27.3 ± 0.2 degrees, 28.2 ± 0.2 degrees, 28.6 ± 0.2 degrees, 29.2 ± 0.2 degrees, 30.4 ± 0.2 degrees, 31.0 ± 0.2 degrees, 32.4 ± 0.2 degrees, 32.7 ± 0.2 degrees and 35.4 ± 0.2 degrees.

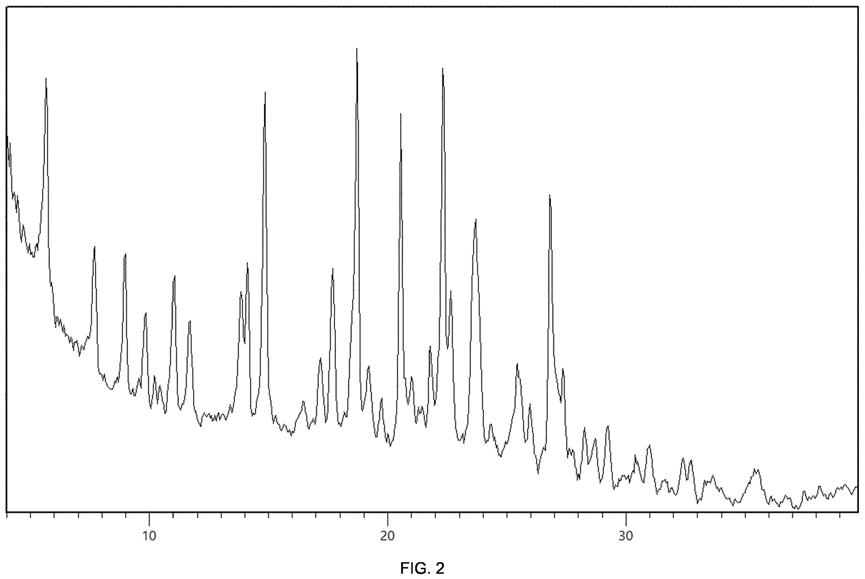

In even more preferred embodiments of the first aspect of the invention, the powder X-ray diffraction spectrum of the crystalline form beta is substantially as shown in Figure 2.

In further preferred embodiments of the first aspect of the invention, the crystalline form beta is further characterized in that it presents a diagram of Differential Scanning Calorimetry which comprises a first endothermic peak with an onset temperature of about 160 °C ± 2 °C, a second endothermic peak with an onset temperature of about 186 °C ± 2 °C, a third endothermic peak with an onset temperature of about 263 °C ± 2 °C and a fourth endothermic peak with an onset temperature of about 280 °C ± 2 °C.

In further preferred embodiments of the first aspect of the invention, in the diagram of Differential Scanning Calorimetry of the crystalline form beta, the first endothermic peak presents an associated heat of about - 1 J/g ± 0.2 J/g, the second endothermic peak presents an associated heat of about - 117 J/g ± 2 J/g, the third endothermic peak presents an associated heat of about - 5 J/g ± 1 J/g and/or the fourth endothermic peak presents an associated heat of about - 29 J/g ± 2 J/g.

In further preferred embodiments of the first aspect of the invention, the crystalline form beta is further characterized in that the thermogravimetric analysis measured between 30 °C and 300 °C with a heating ramp of 10 °C per minute presents a weight loss of about 5% ± 0.2% between 57 °C and 167 °C and, optionally, a further weight loss of about 13% ± 0.5% between 167 °C and 275 °C.

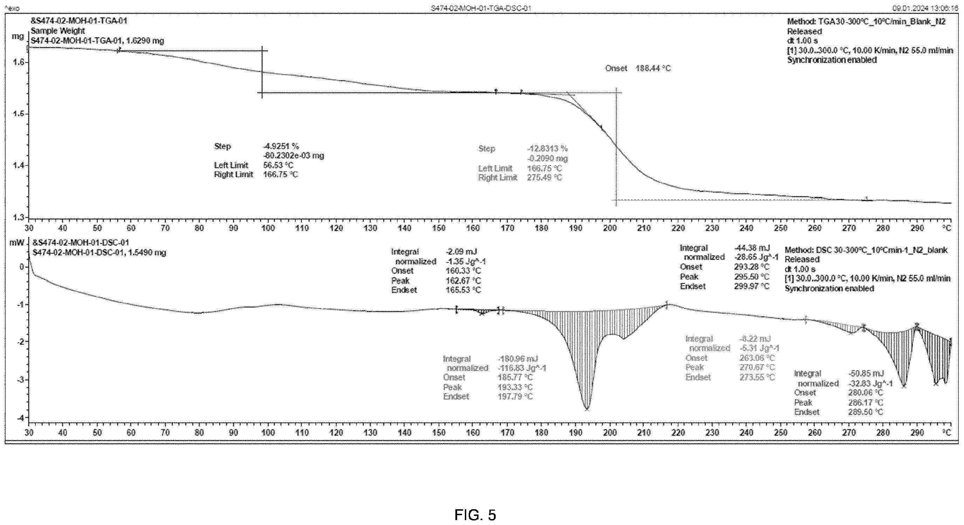

In further preferred embodiments of the first aspect of the invention, the crystalline form beta is one having substantially the thermal gravimetric analysis and Differential Scanning Calorimetry analysis of Figure 5.

In other preferred embodiments of the first aspect of the invention, the powder X-ray diffraction spectrum of the crystalline form gamma measured using a CuKα radiation further comprises diffraction peaks at least at the following 2 θ values of 8.9 ± 0.2 degrees, 23.0 ± 0.2 degrees; 24.2 ± 0.2 degrees; 26.8 ± 0.2 degrees; 27.1 ± 0.2 degrees and 28.4 ± 0.2 degrees.

In even more preferred embodiments of the first aspect of the invention, the powder X-ray diffraction spectrum of the crystalline form gamma measured using a CuKα radiation further comprises diffraction peaks at one or more, preferably all, of the following 2 θ values of 7.6 ± 0.2 degrees, 9.7 ± 0.2 degrees, 10.3 ± 0.2 degrees, 10.8 ± 0.2 degrees, 11.1 ± 0.2 degrees, 11.5 ± 0.2 degrees, 13.8 ± 0.2 degrees 14.7 ± 0.2 degrees, 15.0 ± 0.2 degrees, 15.3 ± 0.2 degrees, 15.7 ± 0.2 degrees, 16.0 ± 0.2 degrees, 16.3 ± 0.2 degrees, 16.9 ± 0.2 degrees, 17.9 ± 0.2 degrees, 18.1 ± 0.2 degrees, 19.2 ± 0.2 degrees, 19.5 ± 0.2 degrees, 21.3 ± 0.2 degrees, 23.5 ± 0.2 degrees, 23.8 ± 0.2 degrees, 24.7 ± 0.2 degrees, 25.3 ± 0.2 degrees, 27.6 ± 0.2 degrees, 28.7 ± 0.2 degrees, 29.4 ± 0.2 degrees, 30.3 ± 0.2 degrees, 31.1 ± 0.2 degrees, 31.3 ± 0.2 degrees, 32.5 ± 0.2 degrees, 32.8 ± 0.2 degrees and 33.5 ± 0.2 degrees.

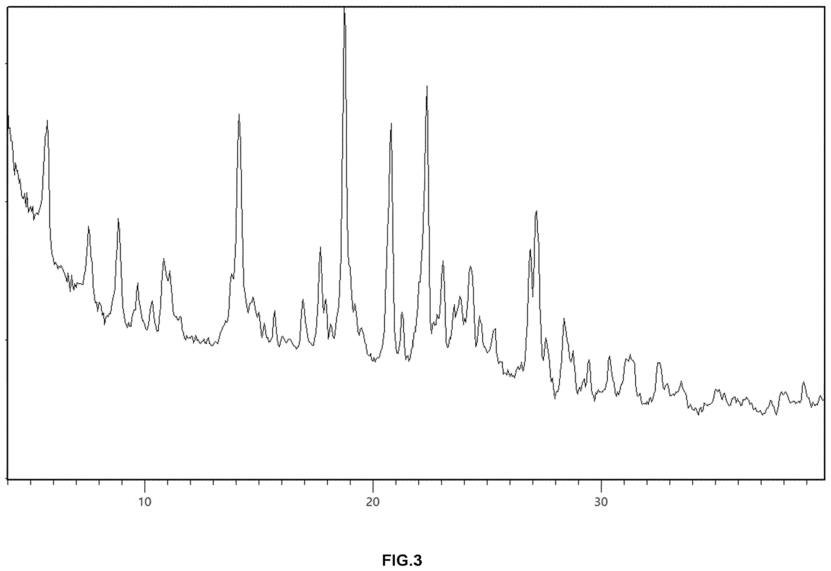

In even more preferred embodiments of the first aspect of the invention, the powder X-ray diffraction spectrum of the crystalline form gamma is substantially as shown in Figure 3.

As mentioned above, the second aspect of the invention relates to a pharmaceutical composition comprising a therapeutically effective amount of the crystalline form of Avatrombopag maleate having the crystal form alpha as defined in the first aspect of the invention and a pharmaceutically acceptable excipient.

The pharmaceutical compositions disclosed herein can be formulated for oral, topical, mucosal (e.g., nasal, pulmonary, sublingual, vaginal, buccal, or rectal), parenteral (e.g., subcutaneous, intravenous, bolus injection, intramuscular, or intraarterial), or transdermal administration to a patient. Examples of dosage forms include, but are not limited to: tablets; caplets; capsules, such as soft elastic gelatin capsules; cachets; troches; lozenges; dispersions; suppositories; ointments; cataplasms (poultices); pastes; powders; dressings; creams; plasters; solutions; patches; aerosols (e.g., nasal sprays or inhalers); gels; liquid dosage forms suitable for oral or mucosal administration to a patient, including suspensions (e.g., aqueous or non-aqueous liquid suspensions, oil-in-water emulsions, or a water-in-oil liquid emulsions), solutions, and elixirs; liquid dosage forms suitable for parenteral administration to a patient; and sterile solids that can be reconstituted to provide liquid dosage forms suitable for parenteral administration to a patient.

The term "pharmaceutically acceptable excipient" refers to a carrier, diluent, or adjuvant which is administered with the active ingredient. Examples of pharmaceutically acceptable excipients for the oral dosage pharmaceutical compositions of the invention are conventional excipients known in the art such as binding agents, for example, syrup, gum arabic, gelatin, sorbitol, tragacanth, crospovidone or polyvinylpyrrolidone; fillers, for example, lactose, mannitol, xylitol, sorbitol, sucrose, corn starch, calcium phosphate, sorbitol, glycine, dextrose, maltodextrin, dextran, dextrin, modified starches; glidants and tablet lubricants, for example magnesium stearate, calcium stearate, stearic acid, zinc stearate, glyceryl monostearate, glyceryl palmitostearate, hydrogenated castor oil, colloidal silicon dioxide, silicon dioxide, anhydrous colloidal silicon, glycerine, hydrogenated vegetable oil, mineral oil, polyethylene glycol, sodium benzoate, sodium lauryl sulfate, sodium stearyl fumarate or talc; disintegrants, for example, starch, polyvinylpyrrolidone, starch sodium glycolate, crospovidone, microcrystalline cellulose, hydroxypropyl cellulose or sorbitan fatty acid esters; pharmaceutically acceptable wetting agents such as sodium lauryl sulfate; water solubilizing aids such as urea, betaine monohydrate, potassium sulfate, potassium acetate, mannitol; alkalinizing agents such as potassium carbonate, sodium carbonate, sodium bicarbonate, trisodium phosphate, tripotassium phosphate, trisodium citrate, tripotassium citrate; sweeteners such as saccharin sodium, sodium cyclamate and aspartame; flavoring agents such as menthol and peppermint oil.

Pharmaceutical compositions of the invention may be administered parenterally, orally or topically, preferably by oral route.

Preferred pharmaceutical excipients comprised in the pharmaceutical composition of the second aspect of the invention are selected from the group consisting of lactose, microcrystalline cellulose, crospovidone, colloidal silicon dioxide, magnesium stearate and mixtures thereof. This is particularly the case when the composition of the second aspect of the invention is a dosage form for oral administration.

Oral dosage forms may further comprise an external coating. Preferred coatings are those suitable for releasing the drug in the gastrointestinal tract. Suitable coatings are known in the art and will become apparent to the skilled person upon reduction of practice of the invention on the basis of common general knowledge.

In a preferred embodiment, the pharmaceutical composition of the second aspect of the invention is in a form suitable for oral administration, either solid or liquid. Suitable dosage forms for oral administration may be tablets, caplets, capsules, cachets, trochets, lozenges, granules, sachets, solutions, suspensions, emulsions and pastes. Preferably the dosage form is selected from the group consisting of solid dosage forms for oral administration, such as tablets and capsules.

The above formulations may be prepared using standard methods such as those described or contemplated in the European and US pharmacopoeias and similar reference texts.

It is also contemplated that these dosage forms are packaged. Packaged forms of dosage forms are well known in the art and will become readily apparent to the skilled person upon reduction to practice of the invention using common general knowledge.

Such packaging includes, among others, blister packs, bottes, strip packs, sachets, foil pouches, tubes, jars, pump dispensers, sachets, pouches, vials, ampoules, oral syringes, pre-filled syringes, IV bags, metered-dose inhalers, spray bottles and dry powder inhalers.

In a preferred embodiment of the invention, the pharmaceutical composition of the second aspect of the invention is in the form of tablet compositions for oral administration, said tablets being stabilized by a moisture barrier created by means of packaging the tablet in a material for blister pack. This allows increasing the shelf life of the packaged solid form. The value of water vapor transmission rate (WVTR value) is a known parameter to express the passage of water vapor through the packaging material in order to control the required quality and shelf life. The WVTR value is generally determined by the thickness of the film forming the barrier and the type of material employed for forming the film. Such WVTR values may be determined according to ASTM F1249. in said embodiment of the second aspect of the invention, the tablet compositions are preferably stabilized by a moisture barrier with a WVTR of less than 0.35 g/m²/day at 38°C/90% RH; preferably of less than 0.2 g/m²/day at 38°C/90% RH; more preferably of less than 0.1 g/m²/day at 38°C/90% RH; even more preferably of less than 0.05 g/m²/day at 38°C/90% RH and even more preferably of less than 0.01 g/m²/day at 38°C/90% RH. Particularly, the blister pack material is Cold Form Foil (CFF). Cold Form Foil (CFF), also known as Alu-Alu foil, has a WVTR value of 0.005 g/m²/day at 38°C/90% RH.
in a preferred embodiment of the invention, the pharmaceutical composition of the second aspect of the invention is in the form of tablet compositions for oral administration. In another preferred embodiment, the tablets are packaged in a blister pack, said blister pack being selected from the group comprising of aluminium/aluminium, aluminium/PVC, aluminium/PVDC, aluminium/PVC/PVDC, aluminium/PCTFE and aluminium/ACLAR. in a most preferred embodiment, the tablets are packaged in a blister pack, said blister pack being selected from the group comprising of aluminium/aluminium, aluminium/PVC, aluminium/PVDC, aluminium/PVC/PVDC, aluminium/PCTFE and aluminium/ACLAR, wherein said tablets are stabilized by a moisture barrier created by means of packaging the tablet in a material for blister pack, increasing the shelf life of the packaged solid form.

The amount of Avatrombopag maleate in said pharmaceutical composition may be such that it allows for daily administration of Avatrombopag maleate. Thus, the pharmaceutical composition of the second aspect of the invention may comprise 20 mg of Avatrombopag maleate. In further embodiments, the pharmaceutical composition of the second aspect of the invention may comprise 40 mg of Avatrombopag maleate. In other embodiments, the pharmaceutical composition of the second aspect of the invention may comprise 60 mg of Avatrombopag maleate. Preferably, in said embodiments, the pharmaceutical composition is for oral administration.

As mentioned above, the third aspect of the invention relates to the crystalline form of Avatrombopag maleate having the crystal form alpha or pharmaceutical composition according to the second aspect of the invention for use in medicine. Since the solubility of the crystal form alpha in gastric pH is higher than other crystal forms known in the art, it is believed that the therapeutic effect of the crystalline form alpha of Avatrombopag maleate or pharmaceutical composition according to the second aspect of the invention will initiate earlier than with the other solid forms of Avatrombopag maleate disclosed in the art.

The fourth aspect of the invention relates to the crystalline form of Avatrombopag maleate having the crystal form alpha or pharmaceutical composition according to the second aspect of the invention for use in the treatment or prevention of thrombocytopenia or chronic liver disease.

A process for the preparation of a crystalline solid form alpha as defined in the first aspect of the invention also forms part of the invention. As mentioned above, the crystal form alpha of the first aspect of the invention may be obtained from the crystalline form beta; in particular by desolvation of the crystalline form beta. Likewise, the inventors have found that crystalline form beta forms spontaneously from the crystalline form gamma. Alternatively, the inventors have developed a process for the preparation of crystalline form alpha. Thus, the fifth aspect of the invention relates to a process for the preparation of a composition comprising a crystalline form alpha as defined in the first aspect of the invention comprising the steps of:
in a first alternative,
(i) providing a crystalline form of Avatrombopag maleate that is selected from the group consisting of a crystalline form gamma as defined in the first aspect of the invention, a crystalline form beta as defined in the first aspect of the invention and a mixture thereof;
(ii) desolvating the crystalline form of Avatrombopag maleate provided in step (i);
   or; in a second alternative,
(iii) contacting an amount of Avatrombopag with a substantially equimolar amount of maleic acid in methanol to provide a suspension;
(iv) isolating the solid resulting from step (iii); and
(v) drying the solid resulting from step (iv) under vacuum and at a temperature of between 60 °C and 160 °C.

In a preferred embodiment of the first alternative of the fifth aspect of the invention, step (ii) is carried out by heating under vacuum at a temperature of at least 50 °C, preferably of between between 60 °C and 160 °C the crystalline form provided in step (i).

In a preferred embodiment of the first alternative of the fifth aspect of the invention, step (i) comprises contacting amorphous Avatrombopag maleate with methanol. Step (i) may be carried out at any temperature; preferably however, step (i9 is carried out at a temperature of between 20 °C and 65 °C.

In a preferred embodiment of the first alternative of the fifth aspect of the invention, step (i) is carried out at a concentration of amorphous Avatrombopag maleate of between 4 and 8 mg per mL of methanol.

In a preferred embodiment of the first alternative of the fifth aspect of the invention, step (i) comprises the previous step of preparing amorphous Avatrombopag maleate. Processes for the preparation of Avatrombopag maleate are known in the art and have been disclosed for instance in IN 429420 B and WO 2020/044364 A1, which processes are incorporated herein by reference. Preferably, amorphous Avatrombopag maleate is obtainable by:
(i) contacting a suspension of crystalline form C of Avatrombopag maleate in an alcohol solvent such as ethanol with an aqueous solution of alkaline hydroxide, such as sodium hydroxide, so as to dissolve any suspended solid;
(ii) adding an aqueous solution of maleic acid to the solution produced in (i) and isolating the resulting solid; and
(iii) suspending the solid obtained in (ii) in water and isolating the resulting solid.
The concentration of Avatrombopag maleate in step (i) is of between 0.05 g/mL and 0.2 g/mL. Preferably, the amount of alkaline hydroxide added to said solution is such that the ratio of alkaline hydroxide to Avatrombopag is of at least 8 to 1; more preferably of at least 10 to 1. In step (ii), maleic acid is added in a large excess, preferably such that the ratio of maleic acid to Avatrombopag is of at least 8 to 1; more preferably of at about 13 to 1. Also preferably, step (iii) of the process for the preparation of amorphous Avatrombopag maleate is carried out a multiplicity of times.

The inventors have found that the process of the first alternative of the fifth aspect of the invention is particularly suitable for the preparation of crystalline forms alpha and beta; step (i) being particularly suitable for the production of the crystalline form beta, optionally mixed with crystalline form gamma.

In a preferred embodiment of the second alternative of the fifth aspect of the invention, the concentration of Avatrombopag in step (iii) is between 10 and 100 mg per mL of methanol; more preferably of about 40 mg of Avatrombopag per mL.

In a preferred embodiment of the second alternative of the fifth aspect of the invention, step (iv) is carried out by filtration.

In a preferred embodiment of the second alternative of the fifth aspect of the invention, step (v) is carried out at temperature of between 70 °C and 90 °C; preferably of about 75 °C or about 80 °C. The duration of step (v) may determine the nature of the obtained crystalline form.

In some embodiments, the process of the second alternative of the fifth aspect of the invention produces a mixture of crystal forms gamma and alpha. It is however possible to produce a crystalline form alpha from said mixture by submitting said mixture to step (v) as defined in the second alternative of the fifth aspect of the invention.

Throughout the description and claims the word "comprises" and variations of the word, are not intended to exclude other technical features, additives, components or steps. Furthermore, the word "comprise" encompasses the cases of "consist of" and "consists essentially of". Additional objects, advantages and features of the invention will become apparent to those skilled in the art upon examination of the description or may be learned by practice of the invention. The following examples are provided by way of illustration, and they are not intended to be limiting of the present invention.

### EXAMPLES

### Examples

### General considerations

Avatrombopag maleate used herein as starting material corresponds to crystalline solid form C, obtainable according to the process disclosed in WO 2013/018362 A1, the content of which is incorporated herein by reference.

The free base of Avatrombopag used herein as starting material may be obtained from commercial sources or according to a synthetic procedure such as the one disclosed in WO 2003/062233 A1, the content of which is disclosed herein by reference.

The Powder X-Ray diffraction (PXRD) patterns were acquired on a Bruker D8 Advance Series 2Theta/Theta powder diffraction system using CuKα1-radiation operated at 40 kV and 40 mA in transmission geometry. The system is equipped with a VÅNTEC-1 single photon counting PSD, a Germanium monochromator, a ninety positions auto changer sample stage, fixed divergence slits and a radial soller. Programs used: Data collection with DIFFRAC plus XRD Commander V.2.5.1, and evaluation with High Score Plus 4.9 (Malvern PANalytical B.V.). Measurement conditions: The samples were measured in a range from 4 to 40° in 2θ in a 6 or 30 minutes measurement using an angular step of 0.05° and a time per step of 432 or 2198 s. Sample preparation: 20 mg of non-manipulated sample were prepared in standard sample holders using two foils of polyacetate

The differential Differential Scanning Calorimetry (DSC) analysis were recorded in a Mettler Toledo DSC822e calorimeter. Programs used: Data collection and evaluation with software STARe. Measurement conditions: The samples were heated under dry nitrogen (flow rate: 50 mL/min) at 10 °C/min from 30 to 300 °C. Sample preparation: Approximately 1-4 mg of sample were weighed (using a MX5 Mettler Toledo microbalance) into 40 µL aluminium crucibles with a pinhole lid.

Thermogravimetric Analysis (TGA) were recorded in a Mettler Toledo TGA/DSC 3+ with a balance XP1 type. Programs used: Data collection and evaluation with software STARe. Measurement conditions: The samples were heated under dry nitrogen (flow rate: 10 mL/min) at 10 °C/min from 30 to 300 °C. Sample preparation: Approximately 1-4 mg of sample were weighed (using a MX5 Mettler Toledo microbalance) into 40 µL aluminium crucibles with a pinhole lid.

Dynamic Vapor Sorption Analysis (DVS) were performed in a Mettler Toledo TGA / DSC 1 LF instrument equipped with a LF SDTA FRS2 sensor and coupled with a Modular Humidity Generator MHG 32. Data collection and evaluation was done with STARe software. Measurement conditions: The samples were analysed following a humidity cycle from 20%RH up to 80%RH, down to 3%RH, up to 90 and down to 3%RH in 10% steps of 30 minutes at 25°C. Sample preparation: Approximately 5-10 mg of sample were weighed (using a MX5 Mettler Toledo microbalance) into 150 µL platinum crucibles without lid.

Proton nuclear magnetic resonance analyses (¹H NMR spectroscopy) were recorded in a Bruker 300 NMR spectrometer, equipped with a z gradient 5 mm BBO (Broadband Observe) probe with ATM and an automatic autosampler. Measurement conditions: The samples were analysed at room temperature. Sample preparation: Approximately 2-5 mg of sample were dissolved in 0.7 mL of deuterated solvent (dimethylsulfoxide-d₆).

Chromoatrographic analyses were performed with an HPLC Agilent Technologies 1200 series instrument. The method used comprises:
- Column: Zorbax SB-C18 2.1x50 mm, 1.8 micron.
- Mobile phase: MOH/H2O 85:15 + 0.1% TFA isocratic.
- Flux: 1 mL/min.
- Wavelength: 210 nm.
- Injection volume: 5 µL.
Data collection and evaluation was done with OpenLab CDS ChemStation.

### Screening of polymorphism of Avatrombopag maleate

### Preparative Example: preparation of amorphous Avatrombopag maleate

Avatrombopag maleate (2 g, 2.6 mmol) was suspended in ethanol (18 mL). Sodium hydroxide (3M in water, 9 mL) was added, providing a clear solution. Maleic acid (4 g, 34.5 mmol, ~13 equivalents) dissolved in water (40 mL) was added over the solution, and the obtained suspension was stirred for 15 minutes. The solid was filtered off, suspended again in water (40 mL) and stirred for 15 minutes. This process was repeated twice. Finally, the solid was filtered off and dried in vacuum at 50 °C for 2 hours

### General Procedure A: Slurry at room temperature

Amorphous Avatrombopag maleate (20 mg) was suspended at room temperature in 3 mL of the corresponding solvent, as shown in Table 1. The mixture was stirred for 24 hours, and afterwards the solid was separated, dried in vacuum (2-5 mbar) for 2 hours at room temperature and analyzed.

### General Procedure B: Slurry at room temperature

Amorphous Avatrombopag maleate (20 mg) was suspended at reflux or at a maximum of 75°C in 2 mL of the corresponding solvent, as shown in Table 1. The mixture was stirred for 2 hours, and afterwards the solid was separated, dried in vacuum (2-5 mbar) for 2 hours at room temperature and analyzed.

### General Procedure C: Spontaneous crystallization

Amorphous Avatrombopag maleate (20 mg) was dissolved in the minimum amount of the corresponding solvent as shown in Table 1 at room temperature. After a short time, spontaneous crystallization was produced. After isolation of the crystals, the resulting solid was analysed.

### General Procedure D: Evaporation of solvent at room temperature

Amorphous Avatrombopag maleate (20 mg) was dissolved in the minimum amount of the corresponding solvent. The solutions were left to evaporate at room temperature

### General Procedure E: Slow cooling crystallization

Amorphous Avatrombopag maleate (20 mg) was dissolved in the minimum amount of the corresponding solvent at reflux or a maximum of 75°C. The obtained solutions were slowly cooled to room temperature. Afterwards, the solid was separated, dried in vacuum (2-5 mbar) for 2 hours at room temperature and analyzed.

### General Procedure F

200 mg of amorphous Avatrombopag maleate in 10 mL of the corresponding solvent, as shown in Table 1, were slurried for 15h. at room temperature. Afterwards, the solid was separated and analyzed
Table 1 below summarizes the obtained crystal forms identified during the screening.

**Table 1**

| Procedure | solvent | Obtained solid form | Procedure | solvent | Obtained solid form |
|---|---|---|---|---|---|
| C | N,N-Dimethylamide | C10 | A | Ethanol | A |
| C | 2-ethoxyethanol | C11 | A | Isopropanol | A |
| A | tert-Butanol | C12 | A | n-Butanol | A |
| C | Cyclohexanone | C12 | A | 1-Propanol | A |
| A | Methyl tert-butyl ether | beta | B | n-Butanol | A |
| B | Methyl ethyl ketone | C15 | B | 2-Butanol | B |
| B | Water | C16 | B | Ethanol | B |
| E | tetrahydrofuran | C17 | B | Isopropanol | B |
| | | | B | 1-Propanol | B |
| B | Chloro | C19 | B | tert-Butanol | B |
| A | 2-Butanol | C2 | B | Ethyl acetate | B |
| A | Ethyl acetate | C3 | B | Isobutyl acetate | B |
| A | Propyl acetate | gamma | B | Isopropyl acetate | B |
| A | Methanol | | B | Propyl acetate | B |
| B | Methanol | | B | Ethyl ate | B |
| A | Methyl acetate | C5 | B | Diisopropyl ether | B |
| A | 1,4-Dioxane | | B | 1,2-Dimethoxyethane | B |
| A | Tetrahydrofuran | C6 | B | Dimethoxymethane | B |
| A | Dimethoxymethane | | B | Methyl tert-butyl ether | B |
| A | Methyl ethyl ketone | C7 | B | 1,4-Dioxane | B |
| A | 1,2-Dimethoxyethane | | B | Methyl isobutyl ketone | B |
| A | Methyl isobutyl ketone | C8 | B | 3-Pentanone | B |
| A | Dimethyl carbonate | | B | Toluene | B |
| A | 3-Pentanone | C9 | B | Anisole | B |
| A | Chloro | | B | Chlorobenzene | B |
| B | Dichloromethane | | B | 1,1,1-Trichloroethane | B |
| D | N,N-Dimethylacetamide | M1 | B | Dimethyl carbonate | B |
| D | Dimethylsulfoxide | M3 | A | Ethyl ate | C |
| A | Isobutyl acetate | C1 + C6 | A | Acetone | C |
| A | Isopropyl acetate | C1 + C7 | A | Nitromethane | C |
| A | Dichloromethane | C9 + C | B | Acetone | C |
| A | Acetonitrile | | B | Nitromethane | C |
| B | Acetonitrile | | F | Methyl acetate | C21 |
| F | Ethyl acetate | C22 | F | Methyl ethyl ketone | C21 |
| F | n-butanol | B | F | Tetrahydrofuran | C22 |

Forms A, B and C were identified by their characterizing information (i.e PXRD and/or DSCT/TGA) disclosed in WO 2013/018362 A1. Likewise, forms M1 and M3 were identified by their characterizing information (i.e PXRD and/or DSCT/TGA) disclosed in WO 2020/044364 A1.

Table 2 below lists the 2θ values (in degrees) of the main and secondary peaks of the PXRD patterns of the identified crystalline forms when measured using CuKα radiation.

**Table 2**

| Solid form | Main peaks (2θ values) | Intermediary peaks (2θ values) |
|---|---|---|
| Form C10 | 8.4; 11.7; 18.0; 22.1; 24.0; 27.8 | 5.4; 18.6; 19.1; 21.6; 23.7; 25.8 |
| Form C11 | 5.2; 19.2; 19.4; 23.2; 24.8; 25.9 | 7.1; 14.7; 15.8; 16.3; 16.7; 27.2 |
| Form C12 | 4.9; 8.7; 15.0; 18.3; 21.3; 22.8 | 10.0; 17.5; 19.0; 20.0; 21.9; 24.7 |
| Form beta | 5.6; 14.8; 18.7; 20.5; 22.3; 26.8 | 9.0; 11.0; 13.8; 17.7; 22.6; 23.7 |
| Form C15 | 5.9;18.7; 20.5; 22.5; 22.9; 24.6 | 8.6; 16.5; 19.1; 24.9; 27.6; 32.8 |
| Form C16 | 5.8; 12.7; 14.2; 16.2; 20.8; 21.9 | 11.4; 12.2; 14.7; 16.8; 19.9; 21.6 |
| Form C17 | 5.1; 7.1; 10.1; 19.6; 21.8; 23.2 | 14.8; 17.8; 20.3; 23.7; 24.4; 26.5 |
| Form C19 | 5.6; 15.3; 17.2; 19.5; 22.7; 26.3 | 8.6; 9.6; 14.6; 20.8; 23.1; 27.1 |
| Form C2 | 7.1; 17.8; 23.2; 23.7; 24.9; 25.6 | 8.7; 15.4; 16.3; 16.9; 20.7; 26.6 |
| Form gamma | 5.7; 14.1; 17.7; 18.7; 20.7; 22.3 | 8.9; 23.0; 24.2; 26.8; 27.1; 28.4 |
| Form C5 | 4.9; 6.4; 17.7; 18.5; 19.3; 21.3 | 14.3; 16.9; 19.8; 22.6; 25.3; 26.5 |
| Form C6 | 9.7; 19.6; 20.0; 23.1; 24.5; 26.8 | 16.2; 17.6; 18.4; 21.5; 24.1; 27.2 |
| Form C7 | 5.4; 9.2; 12.0; 18.5; 22.1; 23.8 | 8.6; 10.5; 19.6; 21.7; 25.8; 27.7 |
| Form C8 | 5.8; 18.6; 20.5; 22.3; 24.3; 27.3 | 14.1; 17.4; 17.9; 22.7; 26.7; 28.2 |
| Form C9 | 8.9; 16.3; 16.8; 17.1; 19.4; 22.1 | 16.0; 18.0; 19.0; 23.2; 25.1; 28.0 |
| Form C21 | 18.6; 20.5; 22.5; 24.6; 24.9; 27.6 | 5.8; 8.6; 19.1; 22.9; 26.9; 32.7 |
| Form C22 | 8.6; 10.7; 11.0; 14.2; 24.6; 24.9 | 5.8; 13.7; 13.9; 22.9; 24.2; 26.5 |

Table 3 below lists the main peaks observed in DSC analysis as well as the corresponding heat exchange as well as the profile of the TGA analysis for each of the identified solid forms.

**Table 3**

| Solid form | DSC analysis (onset temperature of peaks and exchanged energy) | TG analysis (weight loss as a function of temperature) |
|---|---|---|
| Form C10 | endothermic peaks with onsets at 175°C (-137 J/g) and 295°C (-48 J/g) | Weight losses of 17.2% between 150 and 225°C, and of 2.5% between 225 and 300°C |
| Form C12 | endothermic peaks with onsets at 154°C (-117 J/g), 286°C (-55 J/g) and 294°C (-12 J/g) | Weight loss of 0.7% between 55 and 95°C, 12.4% between 100 and 220°C, and of 8.7% between 220 and 280°C |
| Form beta | endothermic peaks with onsets at 160°C (-1 J/g), 186°C (-117 J/g), 263°C (-5 J/g), 280°C (-33 J/g) and 293°C (-29 J/g) | Weight loss of 5% between 50 and 160°C and 12.8% between 170 and 270°C |
| Form C15 | endothermic peaks with onsets at 124°C (-54 J/g), 240°C (-63 J/g), exothermic peak with onset at 250°C (4 J/g), endothermic peaks with onsets at 280°C (-59 J/g) and 289°C (-8 J/g) | Weight loss of 7.6% between 110 and 160°C and 10.6% between 210 and 275°C |
| Form C16 | endothermic peaks with onsets at 84°C (-38 J/g), 151°C (-6 J/g) and 183°C (-28 J/g), and exothermic peak at 202°C (34 J/g). | Weight losses of 4% between 40 and 130°C and 7.3% between 130 and 260°C |
| form C17 | endothermic peaks with onsets at 173°C (-27 J/g), 197°C (-34 J/g), exothermic peak with onset at 222°C (47 J/g) and endothermic peak with onset at 290°C (-55 J/g) | Weight loss of 18.3% between 100 and 275°C |
| Form C19 | endothermic peaks with onsets at 184°C (-118 J/g), exothermic peak with onset at 210°C (34 J/g) and endothermic peak with onset at 292°C (-47 J/g). | Weight loss of 17.2% between 90 and 300°C |
| Form C2 | endothermic peaks with onsets at 156°C (-14 J/g), 206°C (-84 J/g), exothermic peak at 221°C (10 J/g), endothermic peaks with onsets at 280°C (-56 J/g) and 290°C (-5 J/g). | Weight loss of 19.2% between 95 and 275°C |
| Form C5 | endothermic peak with onset at 143°C (-30 J/g), exothermic peak with onset at 214°C (20 J/g), endothermic peaks with onsets at 255°C (-14 J/g) and 286°C (-42 J/g) | Weight loss of 10.7% between 60 and 175°C, and 10.2% between 176 and 300°C |
| Form C6 | endothermic peaks with onsets at 223°C (-146 J/g), 287°C (-80 J/g) and 295°C (-8 J/g) | Weight loss of 14% between 190 and 260°C |
| Form C7 | endothermic peak with onset at 170°C (-67 J/g), exothermic peak with onset at 206°C (73 J/g) and endothermic peak with onset at 291°C (-43 J/g) | Weight loss of 17.9% between 130 and 300°C |
| Form C8 | endothermic peaks with onsets at 165°C (-93 J/g), 195°C (-14 J/g), exothermic peak with onset at 209°C (13 J/g), endothermic pics with onsets at 281°C (-63 J/g) and 290°C (-4 J/g) | Weight loss of 9.9% between 100 and 185°C, and 10.2% between 185 and 300°C |
| Form C21 | endothermic peaks with onsets at 170°C (-134 J/g), exothermic peak with onset at 209°C (13 J/g), endothermic overlapped peak with onset at 238°C and 291°C (-51 J/g) | Weight loss of 2.7% between 130 and 160°C, 8.5% between 160 and 205°C and of 9% between 205 and 300°C |

The above solid forms C2, C7, C8, C10, C12, C14, C15, C16, C17 and C21 are suspected to be solvate forms, as residual peaks of solvents are typically observed in the corresponding ¹H NMR spectra of said solid forms. The presence of the respective solvents in these solid forms present certain drawbacks with respect to industrial manufacturing and/or regulatory compliance, such that these forms have been discarded for further study.

Remarkably, attempts to reproduce solid forms C2, C6, C9, C19 and C16 were not successful, indicating that these solid forms are not suitable for industrial manufacturing given that industrial manufacturing requires a reproducible and reliable preparation procedure providing constant results. Also, forms C22 and C5 were found to lose crystallinity with time, which indicates instability of the corresponding solid forms. Form C11 was found to be unstable and to convert to C14 with time. Form C9 was only exclusively obtained using chloroform as a solvent, which solvent is not suitable for industrial manufacturing of a drug according to regulatory requirements. These solid forms are consequently not suitable for industrial manufacturing of a drug.

### Preparation of crystalline form alpha

### Procedure 1

Amorphous Avatrombopag maleate (200 mg) was suspended in methanol (10 mL) at room temperature, and stirred for 15 hours, filtered and dried in the air. Analysis of the resulting solid by PXRD indicated that the obtained crystalline form was a mixture of forms beta and gamma. After 24 hours, the solid was analyzed by PXRD again - only form beta was observed. ¹H NMR analysis of said solid form indicated the presence of methanol in the crystalline form. In order to remove the methanol to obtain an anhydrous form, form beta was dried in vacuum for 2 hours at 70°C. After said desolvation step, crystalline forma alpha was obtained.

The results of Procedure 1 show that forms beta and gamma are synthetic precursors of the crystalline form alpha of Avatrombopag maleate, form gamma being spontaneously converted into form beta with time.

### Procedure 2

Avatrombopag free base (200 mg) was suspended in methanol (5 mL) at room temperature, and maleic acid (1 molar equivalent, 36 mg) was added. The obtained suspension was stirred for 18 hours. Afterwards, the solid was filtered off and dried in vacuum (2-5 mbar) at 75°C for 4 hours, thus providing crystalline form alpha.

The process of procedure 2 was repeated at the scale of 1 g of Avatrombopag with a higher concentration of Avatrombopag: Avatrombopag free base (1 g) and maleic acid (1 molar equivalent, 180 mg) were suspended in methanol (10 mL) at room temperature. The obtained suspension was stirred for 18 hours. Afterwards, the solid was filtered off and dried in vacuum (2-5 mbar) at 75°C for 3 hours (1.1 g, 93% yield). Analysis of the resulting solid by PXRD indicated that the obtained crystalline form was a mixture of forms alpha and gamma. After further drying in vacuum at 80 °C for 3 hours, further PXRD analysis indicated that the obtained crystalline form was form alpha.

The results of Procedure 2 show that form beta gamma is a synthetic precursor of the crystalline form alpha of Avatrombopag maleate, form gamma being spontaneously converted into form beta upon drying.

### Characterization of crystalline forms alpha, beta and gamma

### PXRD Analysis

Crystalline forms alpha, beta and gamma were analysed by PXRD analysis using CuKα radiation. Table 4 below provides the list of peaks observed in the PXRD pattern of crystalline form alpha, main peaks are marked as bold and underlined text, while intermediate peaks are marked as underlined text.

**Table 4**

| Angle (2θ) | d Value (Å) | Rel. Intensity (%) |
|---|---|---|
| 6,6 | 13,45 | 10 |
| 7,6 | 11,64 | 14 |
| 9,1 | 9,67 | 41 |
| 10,3 | 8,58 | 31 |
| 10,4 | 8,49 | 13 |
| 10,6 | 8,32 | 14 |
| 11,0 | 8,04 | 7 |
| 12,5 | 7,06 | 5 |
| 13,1 | 6,75 | 26 |
| 14,4 | 6,13 | 16 |
| 14,9 | 5,96 | 18 |
| 15,2 | 5,84 | 16 |
| 16,0 | 5,53 | 55 |
| 16,4 | 5,41 | 6 |
| 16,7 | 5,29 | 15 |
| 17,0 | 5,21 | 29 |
| 17,6 | 5,03 | 30 |
| 18,0 | 4,94 | 12 |
| 18,1 | 4,89 | 20 |
| 18,4 | 4,82 | 42 |
| 19,0 | 4,67 | 59 |
| 19,4 | 4,58 | 10 |
| 19,8 | 4,47 | 24 |
| 20,5 | 4,33 | 35 |
| 21,3 | 4,17 | 18 |
| 22,1 | 4,02 | 15 |
| 22,8 | 3,90 | 100 |
| 23,2 | 3,84 | 43 |
| 23,5 | 3,78 | 21 |
| 23,9 | 3,72 | 13 |
| 24,6 | 3,62 | 10 |
| 25,2 | 3,53 | 17 |
| 26,4 | 3,37 | 14 |
| 26,8 | 3,33 | 17 |
| 27,1 | 3,29 | 20 |
| 27,8 | 3,20 | 4 |
| 28,3 | 3,15 | 35 |
| 29,2 | 3,05 | 7 |
| 30,7 | 2,91 | 3 |
| 31,3 | 2,85 | 4 |
| 31,7 | 2,82 | 11 |
| 32,4 | 2,76 | 4 |
| 34,2 | 2,62 | 4 |
| 34,8 | 2,58 | 3 |
| 35,7 | 2,51 | 1 |
| 36,2 | 2,48 | 2 |
| 36,9 | 2,43 | 1 |
| 37,5 | 2,40 | 2 |
| 38,6 | 2,33 | 5 |
| 39,0 | 2,31 | 3 |

The PXRD pattern of crystalline form alpha is also shown in Figure 1.

Table 5 below provides the list of peaks observed in the PXRD pattern of crystalline form beta, main peaks are marked as bold and underlined text, while intermediate peaks are marked as underlined text.

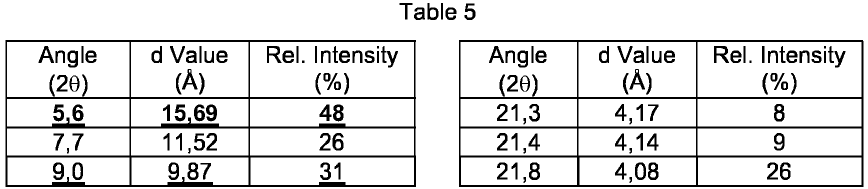

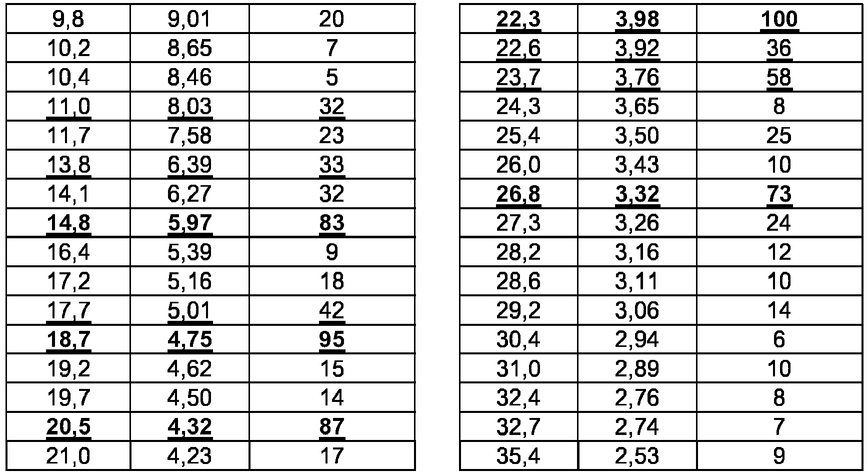

The PXRD pattern of crystalline form beta is also shown in Figure 2.

Table 6 below provides the list of peaks observed in the PXRD pattern of crystalline form beta, main peaks are marked as bold and underlined text, while intermediate peaks are marked as underlined text.

**Table 6**

| Angle (2θ) | d Value (Å) | Rel. Intensity (%) |
|---|---|---|
| 5,7 | 15,55 | 31 |
| 7,6 | 11,69 | 15 |
| 8,9 | 9,98 | 22 |
| 9,7 | 9,12 | 8 |
| 10,3 | 8,57 | 7 |
| 10,8 | 8,16 | 18 |
| 11,1 | 7,96 | 15 |
| 11,5 | 7,71 | 5 |
| 13,8 | 6,42 | 12 |
| 14,1 | 6,26 | 62 |
| 14,7 | 6,01 | 10 |
| 15,0 | 5,90 | 7 |
| 15,3 | 5,80 | 6 |
| 15,7 | 5,65 | 12 |
| 16,0 | 5,52 | 5 |
| 16,3 | 5,42 | 5 |
| 16,9 | 5,23 | 16 |
| 17,7 | 5,01 | 30 |
| 17,9 | 4,95 | 12 |
| 18,1 | 4,88 | 7 |
| 18,7 | 4,73 | 100 |
| 19,2 | 4,62 | 12 |
| 19,5 | 4,54 | 6 |
| 20,7 | 4,28 | 64 |
| 21,3 | 4,17 | 13 |
| 22,3 | 3,99 | 65 |
| 23,0 | 3,86 | 29 |
| 23,5 | 3,78 | 13 |
| 23,8 | 3,73 | 18 |
| 24,2 | 3,67 | 30 |
| 24,7 | 3,60 | 13 |
| 25,3 | 3,52 | 14 |
| 26,8 | 3,32 | 37 |
| 27,1 | 3,28 | 46 |
| 27,6 | 3,23 | 11 |
| 28,4 | 3,14 | 18 |
| 28,7 | 3,11 | 10 |
| 29,4 | 3,03 | 12 |
| 30,3 | 2,94 | 10 |
| 31,1 | 2,88 | 10 |
| 31,3 | 2,85 | 9 |
| 32,5 | 2,75 | 10 |
| 32,8 | 2,73 | 5 |
| 33,5 | 2,68 | 5 |

The PXRD pattern of crystalline form gamma is also shown in Figure 3.

### DSC Analysis and TGA

Crystalline forms alpha and beta were analysed by Differential Scanning Calorimetry and Thermal gravimetric analysis as defined above.

DSC/TG analysis diagram of crystalline form alpha is shown in Figure 4. The DSC diagram exhibits an endothermic peak with onset at 184 °C (-62 J/g), exothermic peaks with onsets at 196 °C (9 J/g) and 210 °C (12 J/g), endothermic peaks with onsets at 244 °C (-56 J/g) and 293 °C (-63 J/g). Also, thermal gravimetric analysis of crystalline form alpha exhibits a weight loss of 4% between 170 and 210 °C and of 9% between 210 and 300 °C.

DSC/TG analysis diagram of crystalline form alpha is shown in Figure 5. The DSC diagram exhibits endothermic peaks with onsets at 160 °C (-1 J/g), 186 °C (-117 J/g), 263 °C (-5 J/g), 280 °C (-33 J/g) and 293 °C (-29 J/g). Also, thermal gravimetric analysis of crystalline form alpha exhibits a weight loss of 5% between 50 and 160 °C and 12.8% between 170 and 270 °C.

Since a 0.5 molar equivalent of methanol is observed on the ¹H NMR spectrum of crystalline form beta, it is believed that crystalline form beta is a hemisolvate of Avatrombopag maleate with methanol. Unlike other solid forms of Avatrombopag maleate described above, the crystalline form alpha is advantageously sufficiently thermally stable for the purposes of industrial manufacturing.

### Hygroscopicity of crystalline form alpha

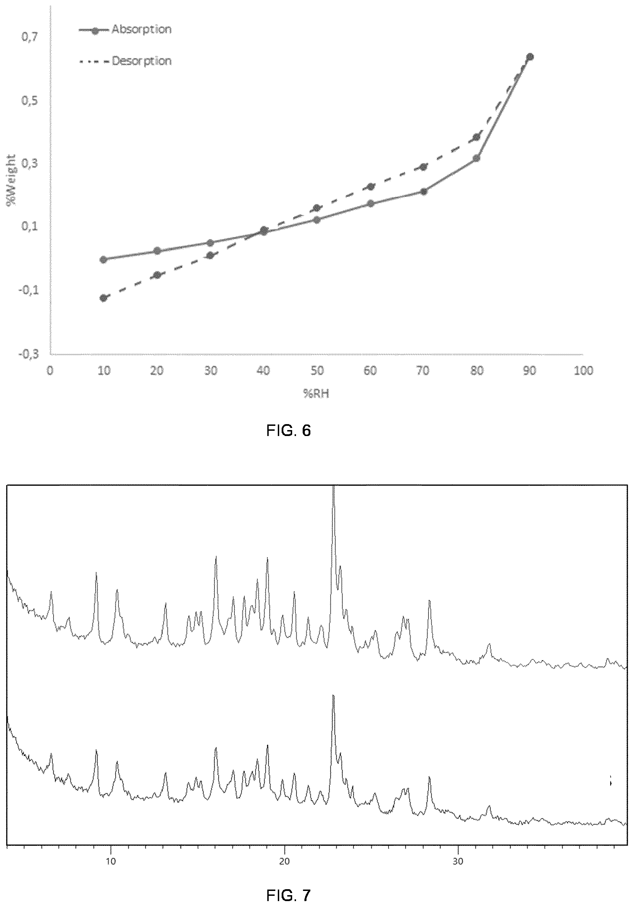

Fig. 6 shows the isotherm of water sorption (plain line) and desorption (dashed line) of crystalline form alpha as measured by Dynamic Vapor Sorption; y axis shows the weight variation of the solid form, expressed as a percentage and x axis shows the relative humidity, also expressed as a percentage.

The results of Figure 6 show that crystalline form alpha is not substantially hygroscopic or exhibits low hygroscopicity, such that it can be advantageously handled with no need for any type of precaution aiming at reducing its exposure to humidity. In particular, Figure 6 shows that the absorption of water by crystalline form alpha causes a gain of weight below 0.7% when the crystalline form alpha is exposed to a relative humidity of 90%. Likewise, Figure 6 shows that the absorption of water by crystalline form alpha causes a gain of weight below 0.4% when the crystalline form alpha is exposed to a relative humidity of 80%. Figure 6 also shows that the absorption of water by crystalline form alpha causes a gain of weight below 0.3% when the crystalline form alpha is exposed to a relative humidity of 70%.

Figure 7 shows PXRD patterns of the crystalline form alpha before and after DVS analysis. Figure 7 shows that the crystalline form alpha preserves its solid form upon exposure to relative humidity as high as 90%.

### Stability of crystalline form alpha

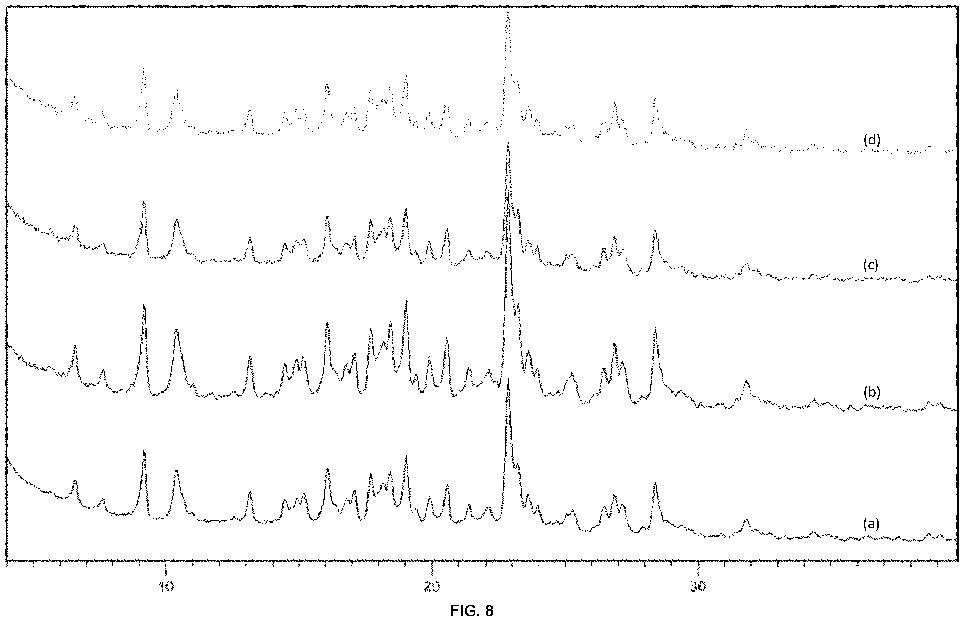

A sample of Crystalline Form Alpha of Avatrombopag maleate was stored for 13 days in a climatic chamber at 40°C and 75% RH, and the solid form was monitored by PXRD. Fig. 8 shows the PXRD patterns of (a) crystal solid form alpha and (b) crystal solid form alpha placed at 40 °C and 75% relative humidity after a period of (b) 1 day, (c) 5 days, (d) 13 days. Figure 8 shows that the crystalline form alpha preserves its solid form upon exposure to relative humidity as high as 70% along with temperatures of 40 °C for long periods of time, i.e. of at least 13 days.

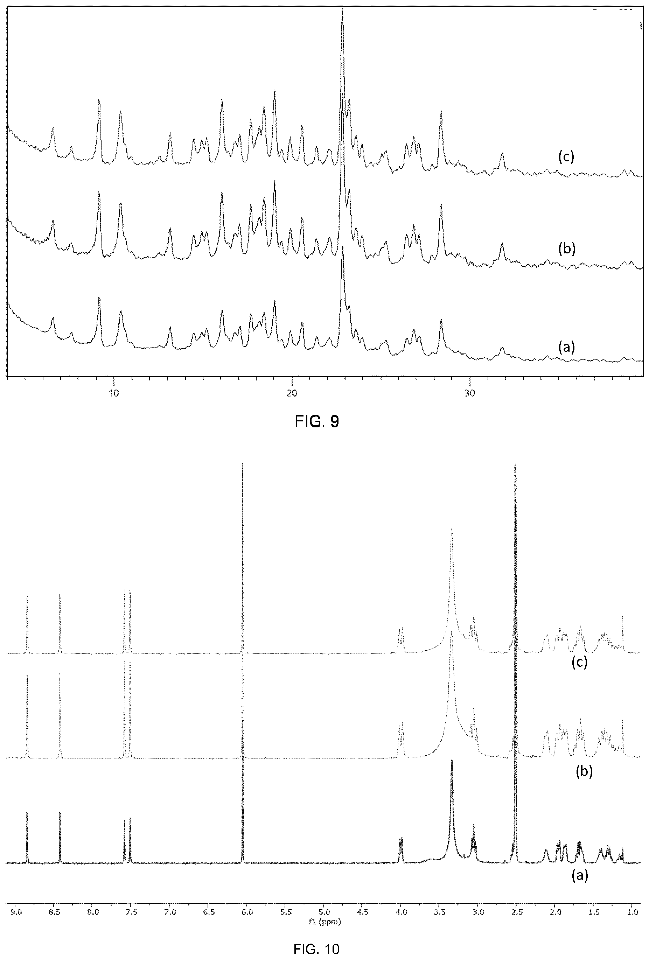

Fig. 9 shows the PXRD patterns of (a) crystal solid form alpha and crystal solid form alpha placed at 90 °C for 24 hours in (b) an open vial or (c) a closed vial.

Fig. 10 shows the ¹H NMR spectra of (a) crystal solid form alpha and crystal solid form alpha placed at 90 °C for 24 hours in (b) an open vial or (c) a closed vial.

Figures 9 and 10 show that that the crystalline form alpha preserves its solid form upon exposure to temperatures as high as 90 °C for periods of time of at least 24 hours. This is consistent with the DSC/TGA analysis of Figure 4.

### Solubility of crystalline form alpha

Thermodynamic solubility of Crystalline Forms Alpha, B and C were compared at 25 °C in a solution of pH 1.2:
- pH 1.2: HCl/KCl buffer. 745 mg of KCl and 17 mL of HCl 1 M were adjusted to a volume of 200 mL in water. The final pH was adjusted using HCl or NaOH 1N.

### a) Preparation of the calibration curve

In order to prepare a calibration curve, two different stock solutions of Avatrombopag maleate in methanol were prepared. From them, five different standard solutions of known concentration were prepared by dilution, which were used to determine de calibration curve for the quantification of Avatrombopag by HPLC analysis.

| Avatrombopag maleate concentration (mg/mL) | Area of the peak (210 nm) |
|---|---|
| 0,541 | 13358 |
| 0,282 | 6691 |
| 0,0541 | 1247 |
| 0,02164 | 462 |
| 0,01128 | 151 |

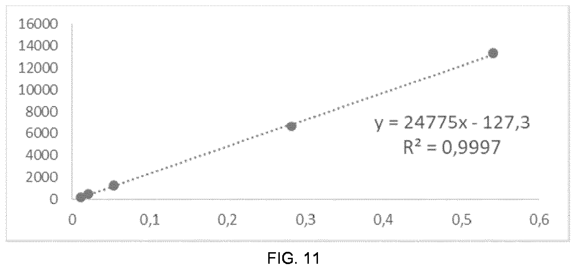

Table 7. HPLC analysis of the standard solutions prepared for the calibration curve. Fig. 11 shows the resulting calibration curve.

### b) Solubility tests.

The solubility of the different forms of Avatrombopag maleate was determined following the general procedure: A sample of the salt (100 - 150 mg) was suspended in the corresponding buffer (3 mL) and the suspension was stirred at room temperature for 24 hours. A sample was taken after this time with a syringe equipped with a syringe filter (Nylon). The samples were analyzed by HPLC to determine the amount of Avatrombopag maleate dissolved in each suspension. This process was performed by duplicate. The duration of this experiment allows reaching equilibrium according to WHO Annex 4 of the Protocol to conduct equilibrium solubility experiments for the purpose of Biopharmaceutics Classification System-based classification of active pharmaceutical ingredients for biowaiver (TRS 1019: Annex 4, WHO Technical Report Series, No. 1019, 2019).

Results are summarized in Table 8, which shows the obtained solubilities of Avatrombopag maleate, expressed in mg/mL after 24 h.

**Table 8**

| pH | time | Form alpha | Form B | Form C |
|---|---|---|---|---|
| 1.2 | 24 h | 0.45 | 0.175 | 0.223 |
| | | 0.44 | 0.135 | 0.323 |

The results of Table 8 show that the crystalline form alpha of Avatrombopag maleate is surprisingly more soluble at gastric pH (pH of 1-2) than other crystalline forms known in the art. In particular, crystalline form alpha is more soluble than crystalline form B or C in the solution having a pH 1.2. An improved solubility at gastric pH is supposed to make an orally administered compound having an improved oral bioavailability. In addition, and as mentioned above, said solid form is thermally stable and poorly hygroscopic, which makes it particularly suitable for manufacturing and handling. The crystalline form alpha thus exhibits at the same time both key characteristics of having a good oral absorption and being suitable for industrial scale up.

## Claims

1. A crystalline form of Avatrombopag maleate that is selected from the group consisting of:
(i) a crystalline form alpha **characterized in that** its powder X-Ray diffraction spectrum measured using a CuKα radiation comprises diffraction peaks at least at the following 2 θ values of 9.1 ± 0.2 degrees; 10.3 ± 0.2 degrees; 16.0 ± 0.2 degrees; 19.0 ± 0.2 degrees; 22.8 ± 0.2 degrees and 28.3± 0.2 degrees;
(ii) a crystalline form beta **characterized in that** its powder X-Ray diffraction spectrum measured using a CuKα radiation comprises diffraction peaks at least at the following 2 θ values of 5.6 ± 0.2 degrees; 14.8 ± 0.2 degrees; 18.7 ± 0.2 degrees; 20.5 ± 0.2 degrees; 22.3 ± 0.2 degrees and 26.8 ± 0.2 degrees;
(iii) a crystalline form gamma **characterized in that** its powder X-Ray diffraction spectrum measured using a CuKα radiation comprises diffraction peaks at least at the following 2 θ values of 5.7 ± 0.2 degrees; 14.1 ± 0.2 degrees; 17.7 ± 0.2 degrees; 18.7 ± 0.2 degrees; 20.7 ± 0.2 degrees; and 22.3 ± 0.2 degrees; and
(iv) a mixture thereof.

2. The crystalline form of Avatrombopag maleate according to claim 1 wherein:
(i) the powder X-ray diffraction spectrum of the crystalline form alpha measured using a CuKα radiation further comprises diffraction peaks at least at the following 2 θ values of 6.6 ± 0.2 degrees, 13.1 ± 0.2 degrees; 17.0 ± 0.2 degrees; 18.4 ± 0.2 degrees; 20.5 ± 0.2 degrees and 23.2 ± 0.2 degrees;
(ii) the powder X-ray diffraction spectrum of the crystalline form beta measured using a CuKα radiation further comprises diffraction peaks at least at the following 2 θ values of 9.0 ± 0.2 degrees, 11.0 ± 0.2 degrees; 13.8 ± 0.2 degrees; 17.7 ± 0.2 degrees; 22.6 ± 0.2 degrees and 23.7 ± 0.2 degrees; and/or
(iii) the powder X-ray diffraction spectrum of the crystalline form gamma measured using a CuKα radiation further comprises diffraction peaks at least at the following 2 θ values of 8.9 ± 0.2 degrees, 23.0 ± 0.2 degrees; 24.2 ± 0.2 degrees; 26.8 ± 0.2 degrees; 27.1 ± 0.2 degrees and 28.4 ± 0.2 degrees.

3. The crystalline form of Avatrombopag maleate according to any one of claims 1 to 2 wherein:
(i) the powder X-ray diffraction spectrum of the crystalline form alpha measured using a CuKα radiation is substantially as shown in Figure 1;
(ii) the powder X-ray diffraction spectrum of the crystalline form beta measured using a CuKα radiation is substantially as shown in Figure 2; and/or
(iii) the powder X-ray diffraction spectrum of the crystalline form gamma measured using a CuKα radiation is substantially as shown in Figure 3.

4. The crystalline form of Avatrombopag maleate according to any one of claims 1 to 3 wherein:
(i) the crystalline form alpha is further **characterized in that** it presents a diagram of Differential Scanning Calorimetry which comprises a first endothermic peak with an onset temperature of about 184 °C ± 2 °C, a second endothermic peak with an onset temperature of about 244 °C ± 2 °C and a third endothermic peak with an onset temperature of about 293 °C ± 2 °C; preferably, said diagram of Differential Scanning Calorimetry further comprises a first exothermic peak with an onset temperature of about 196 °C ± 2 °C and a second exothermic peak with an onset temperature of about 210 °C ± 2 °C; and/or
(ii) the crystalline form beta is further **characterized in that** it presents a diagram of Differential Scanning Calorimetry which comprises a first endothermic peak with an onset temperature of about 160 °C ± 2 °C, a second endothermic peak with an onset temperature of about 186 °C ± 2 °C, a third endothermic peak with an onset temperature of about 263 °C ± 2 °C and a fourth endothermic peak with an onset temperature of about 280 °C ± 2 °C.

5. The crystalline form of Avatrombopag maleate according to claim 4 wherein:
(i) in the diagram of Differential Scanning Calorimetry of the crystalline form alpha, the first endothermic peak presents an associated heat of about - 62 J/g ± 2 J/g, the second endothermic peak presents an associated heat of about - 56 J/g ± 2 J/g and/or the third endothermic peak presents an associated heat of about - 63 J/g ± 2 J/g; preferably, when said diagram comprises a first and a second exothermic peak, the first exothermic peak presents an associated heat of about 9 J/g ± 2 J/g and the second exothermic peak presents an associated heat of about 12 J/g ± 2 J/g; and/or
(ii) in the diagram of Differential Scanning Calorimetry of the crystalline form beta, the first endothermic peak presents an associated heat of about -1 J/g ± 0.2 J/g, the second endothermic peak presents an associated heat of about - 117 J/g ± 2 J/g, the third endothermic peak presents an associated heat of about - 5 J/g ± 1 J/g and/or the fourth endothermic peak presents an associated heat of about - 29 J/g ± 2 J/g.

6. The crystalline form of Avatrombopag maleate according to any of claims 1 to 5 wherein:
(i) the crystalline form alpha is further **characterized in that** the thermogravimetric analysis measured between 30 °C and 300 °C with a heating ramp of 10 °C per minute presents a weight loss of about 4% ± 0.2% between 160 °C and 208 °C and, optionally, a further weight loss of about 9% ± 0.2% between 208 °C and 300 °C; and/or
(ii) the crystalline form beta is further **characterized in that** the thermogravimetric analysis measured between 30 °C and 300 °C with a heating ramp of 10 °C per minute presents a weight loss of about 5% ± 0.2% between 57 °C and 167 °C and, optionally, a further weight loss of about 13% ± 0.5% between 167 °C and 275 °C.

7. The crystalline form of Avatrombopag maleate according to any of claims 1 to 6 having a thermodynamic solubility of at least 0.4 mg/mL when a sample of 100 mg of said crystalline form is placed in a solution having a pH of 1.2 for a period of 24 hours.

8. The crystalline form of Avatrombopag maleate according to any of claims 1 to 7 that is the crystalline form alpha.

9. A pharmaceutical composition comprising a therapeutically effective amount of the crystalline form of Avatrombopag maleate according to claim 8 and a pharmaceutically acceptable excipient.

10. Crystalline form of Avatrombopag maleate according to claim 8 or pharmaceutical composition according to claim 9 for use in medicine.

11. Crystalline form of Avatrombopag maleate according to claim 8 or pharmaceutical composition according to claim 9 for use in the treatment or prevention of thrombocytopenia or chronic liver disease.

12. A process for the preparation of a composition comprising a crystalline form alpha as defined in any one of claims 1 to 8 comprising the steps of:
(i) providing a crystalline form of Avatrombopag maleate that is selected from the group consisting of a crystalline form gamma as defined in any one of claims 1 to 3, a crystalline form beta as defined in any one of claims 1 to 6 and a mixture thereof;
(ii) desolvating the crystalline form of Avatrombopag maleate provided in step (i);
or; alternatively,
(iii) contacting an amount of Avatrombopag with a substantially equimolar amount of maleic acid in methanol to provide a suspension;
(iv) isolating the solid resulting from step (iii); and
(v) drying the solid resulting from step (iv) under vacuum and at a temperature of between 60 °C and 160 °C.

13. The process according to claim 12 wherein step (ii) is carried out by heating under vacuum at a temperature of between 60 °C and 160 °C.

14. The process according to any of claims 12 to 13 wherein step (i) comprises contacting amorphous Avatrombopag maleate with methanol.

15. The process according to any of claims 12 to 14 wherein step (i) comprises contacting amorphous Avatrombopag maleate with methanol at a temperature of between 20 °C and 65 °C.
